# EUROPÄISCHE PATENTSCHRIFT

(11) **EP 0 511 165 B1**
(45) Veröffentlichungstag und Bekanntmachung des Hinweises auf die Patenterteilung: **14.08.1996**
(21) Anmeldenummer: 92810285.4
(22) Anmeldetag: 16.04.1992
(51) Int. Cl.: C07D 487/04, C08K 5/34, C09B 57/00

(54) **Neue Diketopyrrolopyrrolverbindungen**
Dicetopyrrolopyrrole compounds
Dicetopyrrolopyrroles

(30) Priorität: 26.04.1991 CH 1250/91
(43) Veröffentlichungstag der Anmeldung: 28.10.1992
(73) Patentinhaber: CIBA-GEIGY AG, 4002 Basel (CH)
(72) Erfinder: Wooden, Gary, Dr., CH-1716 Oberschrot (CH); de Weck, Guy, Dr., CH-4051 Basel (CH); Wallquist, Olof, Dr., CH-1723 Marly (CH)

(56) Entgegenhaltungen:
- EP-A- 0 133 156

## Beschreibung

Die vorliegende Erfindung betrifft neue, durch mindestens einen Aminoalkoxyrest subsituierte Diketopyrrolopyrrolverbindungen und ihre Verwendung zur Verbesserung der coloristischen und rheologischen Eigenschaften von Diketopyrrolopyrrolpigmenten.

Es ist bekannt, dass durch Zugabe eines kleineren Anteils eines durch geeignete Substitution modifizierten Pigments zu einem Pigment, die Eigenschaften des letzteren verbessert werden können. So beschreibt beispielsweise die DE-OS 38 32 064 ein Verfahren zur Herstellung von Pigmentzubereitungen mit verbesserten coloristischen und rheologischen Eigenschaften, durch Perlmahlung von Anthanthron-Rohpigmenten in Gegenwart von als Dispergatoren definierten durch Substitution mit bestimmten Aminoalkylgruppen modifizierten Pigmenten. Aus EP-A 321 919 sind durch einen heterocyclischen Rest über eine Methylengruppe substituierte Pigmente beschrieben, die im Vergleich zu den unsubstituierten verbesserte Flockungsstabilität und rheologische Eigenschaften zeigen. Im US-Patent 4 791 204 werden Diketopyrrolopyrrolzusammensetzungen bestehend aus einem Diketopyrrolopyrrolpigment und einem kleineren Anteil eines modifizierten Diketopyrrols beschrieben, die sich durch verbesserte rheologische Eigenschaften auszeichnen. Bei den modifizierten Diketopyrrolopyrrolen handelt es sich unter anderen um solche, die durch über eine Methylengruppe gebundene cyclische Carboxamid- oder Dicarboximidgruppen substituiert sind. In JP Kokai 91-26767 werden mit aminischen Resten substituierte Diketopyrrolopyrrolverbindungen als Pigmentdispergatoren beschrieben, die zu verbesserten rheologischen Eigenschaften führen. Die aminischen Reste sind dabei jedoch nicht über eine Alkoxybrücke gebunden.

Es sind nun neue durch mindestens einen Aminoalkoxyrest substituierte Diketopyrrolopyrrole gefunden worden, die in einem kleineren Anteil mit Diketopyrrolopyrrolpigmenten vermischt insbesondere in Lacken und Druckfarben zu überraschend guten coloristischen und rheologischen Eigenschaften führen.

Die vorliegende Erfindung betrifft demnach 1,4-Diketopyrrolo-[3,4-c]-pyrrole der Formel 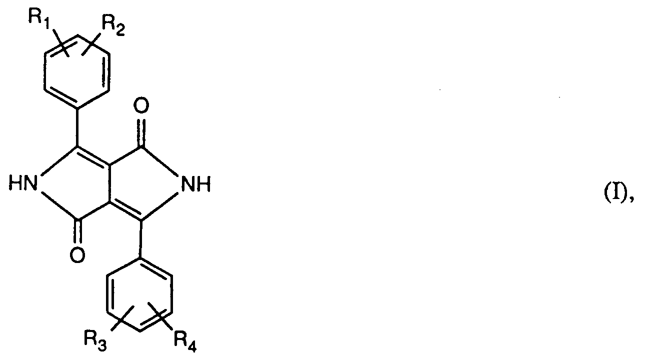 worin R₁, R₂, R₃ und R₄ unabhängig voneinander Wasserstoff, Cl, Br, CH₃, OCH₃, CN oder Phenyl bedeuten und mindestens einer der Reste R₁, R₂, R₃ und R₄ eine Gruppe

-O(CH₂)ₙX oder -O(CH₂CH₂O)ₚCH₂CH₂X

ist, worin
n eine Zahl von 2 bis 12 und
p eine Zahl von 1 bis 3 bedeuten,
X ein unsubstituierter oder ein- oder zweimal durch Methyl substituierter heterocyclischer Rest ausgewählt aus der Gruppe bestehend aus Imidazolyl, Pyrazolyl, Triazolyl, Piperazinyl, pyridinyl, Pyrrolyl, Thiazolyl, Oxazolyl, Benzoxazolyl, Indolyl, Benzthiazolyl, Benzimidazolyl, Benztriazolyl, Morpholinyl, Piperidinyl und Pyrrolidinyl oder eine der Gruppen
-NR₅R₆
oder
-N[(CH₂)ₙ-NR₅R₆]₂ ist, worin
R₅ und R₆ unabhängig voneinander Wasserstoff, C₁-C₆-Alkyl oder C₅-C₆-Cycloalkyl sind.

Bedeuten R₅ und R₆ C₁-C₆-Alkyl, dann handelt es sich z.B. um Methyl, Ethyl, n-Propyl, Isopropyl, n-Butyl, sek.-Butyl, tert.-Butyl, tert.-Amyl und n-Hexyl.

Als C₅-C₆-Cycloalkyl bedeuten R₅ und R₆ Cyclopentyl und bevorzugt Cyclohexyl.

Von besonderem Interesse sind 1,4-Diketopyrrolo-[3,4-c]-pyrrole der Formel I, worin mindestens einer der Reste R₁, R₂, R₃ oder R₄ eine Gruppe

-O(CH₂)ₙX oder -O(CH₂CH₂O)ₚCH₂CH₂X

ist, worin
n und p die oben angegebene Bedeutung haben und
X ein heterocyclischer Rest ausgewählt aus der Gruppe bestehend aus Imidazolyl, Pyrazolyl, Triazolyl, Piperazinyl, Pyridinyl, Pyrrolyl, Thiazolyl, Oxazolyl, Morpholinyl, Piperidinyl und Pyrrolidinyl, oder eine Gruppe
-NR₅R₆
mit der oben angegebenen Bedeutung ist.

Bevorzugt werden 1,4-Diketopyrrolo-[3,4-c]-pyrrole der Formel I, worin einer oder zwei der Reste R₁, R₂, R₃ oder R₄ eine Gruppe

-O(CH₂)ₙX oder -O(CH₂CH₂O)ₚCH₂CH₂X

ist, worin
n eine Zahl von 2 bis 6 und
p die Zahl 1 oder 2 bedeuten und
X ein heterocyclischer Rest ausgewählt aus der Gruppe bestehend aus Imidazolyl, Pyrazolyl, Triazolyl, Piperazinyl, Pyridinyl, Pyrrolyl, Thiazolyl, Oxazolyl, Morpholinyl, Piperidinyl und Pyrrolidinyl,
oder eine Gruppe
-NR₅R₆
bedeutet, worin R₅ und R₆ unabhängig voneinander Wasserstoff, Methyl oder Ethyl bedeuten.

Besonders bevorzugt ist dabei X ein heterocyclischer Rest ausgewählt aus der Gruppe bestehend aus Imidazolyl, pyrazolyl, Morpholinyl, Piperidinyl, Pyrrolidinyl und Triazolyl oder eine Gruppe -NR₅R₆.

Ganz besonders bevorzugt sind 1,4-Diketopyrrolo-[3,4-c]-pyrrole der Formel I, worin einer der Reste R₃ oder R₄ eine Gruppe

-O(CH₂)ₙNR₅R₆ oder -O(CH₂)ₙX

ist, worin n eine Zahl von 2 bis 4 bedeutet und R₅ und R₆ gleich sind und Wasserstoff, Methyl oder Ethyl und X Morpholinyl, Piperidinyl oder Pyrrolidinyl bedeuten.

Wie bereits erwähnt ist gefunden worden, dass die Beimischung eines kleineren Anteils einer erfindungsgemässen Diketopyrrolopyrrolverbindung der Formel I zu einem Diketopyrrolopyrrolpigment eine ganz überraschende Verbesserung der coloristischen und rheologischen Eigenschaften des Letzteren zur Folge hat.

Ein weiterer Gegenstand der Erfindung sind demnach Pigmentzusammensetzungen enthaltend
a) 80-99,9 Gew.% mindestens eines 1,4-Diketopyrrolo-[3,4-c]-pyrrols der Formel I, worin R₁, R₂, R₃ und R₄ unabhängig voneinander Wasserstoff, Cl, Br, CH₃, OCH₃, CN oder Phenyl bedeuten, und
b) 0,1-20 Gew.% mindestens eines 1,4-Diketopyrrolo-[3,4-c]-pyrrols der Formel I, gemäss der unter a) angegebenen Definition, bei dem mindestens einer der Reste R₁, R₂, R₃ und R₄ eine Gruppe

   -O(CH₂)ₙX oder -O(CH₂CH₂O)ₚCH₂CH₂X

   mit der oben angegebenen Bedeutung ist.

Die Diketopyrrolopyrrole gemäss der Komponente a) der erfindungsgemässen Stoffzusammensetzungen stellen bekannte Produkte dar und können beispielsweise gemäss den in den US-Patenten 4 579 949 und 4 749 795 beschriebenen Verfahren hergestellt werden.

Die Diketopyrrolopyrrole der Formel I können in Analogie zu allgemein bekannten Verfahren hergestellt werden, z.B. durch Umsetzung eines Bemsteinsäurediesters mit einem Nitril oder einem Gemisch von Nitrilen der Formeln 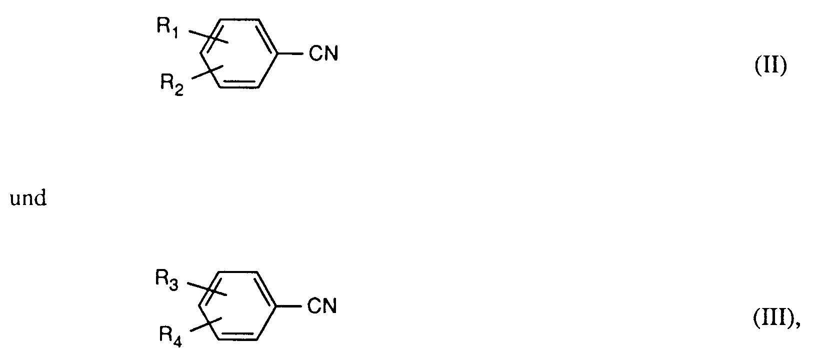 wie beispielsweise aus den US-Patenten 4 579 949 und 4 720 305 bekannt, oder insbesondere im Falle der bevorzugten Verbindungen der Formel I, worin einer der Reste R₃ oder R₄ eine Gruppe

-O(CH₂)ₙX oder -O(CH₂CH₂O)ₚCH₂CH₂X

bedeutet, durch Umsetzung eines Pyrrolinons der Formel 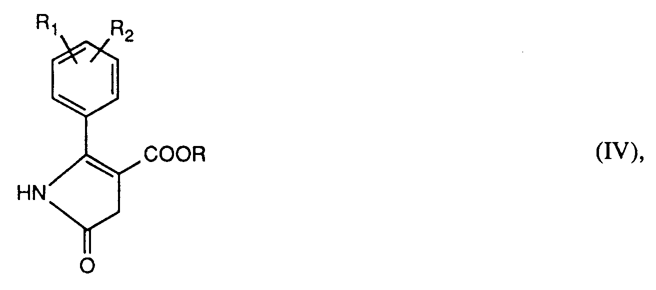 oder eines Enamins der Formel 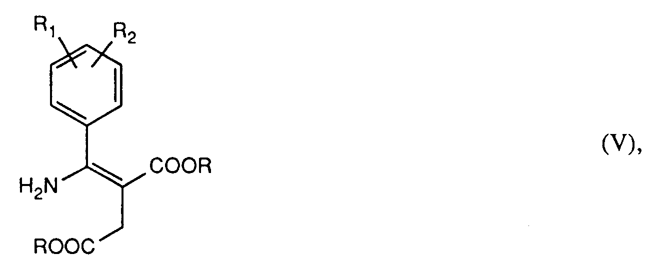 mit einem Nitril oder einem Gemisch von Nitrilen der Formel III, wie z.B. aus den US-Patenten 4 659 775 und 4 749 795 bekannt ist.

R₁, R₂, R₃ und R₄ haben in den Formeln II, III, IV und V die oben angegebene Bedeutung, wobei mindestens einer dieser Reste und bevorzugt einer von R₃ und R₄ eine Gruppe

-O(CH₂)ₙX oder -O(CH₂CH₂O)ₚCH₂CH₂X

mit der oben angegebenen Bedeutung sein muss.

R in den Formeln IV und V bedeutet Niederalkyl, z.B. Methyl, Ethyl, n-Propyl, Isopropyl, n-Butyl, sek.-Butyl, tert.-Butyl und tert.-Amyl. Bevorzugt sind Methyl und Ethyl.

Bei den Verbindungen der Formeln II, III, IV und V handelt es sich um bekannte Verbindungen. Sollten einige davon noch neu sein, so können sie in Analogie zu allgemein bekannten Methoden hergestellt werden.

Die erfindungsgemässen Pigmentzusammensetzungen können z.B. durch folgende allgemein bekannte Methoden hergestellt werden:
- Direkt in der Synthese, durch Umsetzung eines Bernsteinsäurediesters mit verschiedenen Nitrilen der Formeln II und III, wie z.B. aus dem US-Patent 4 720 305 bekannt, wobei das keine Gruppe

   -O(CH₂)ₙX oder -O(CH₂CH₂O)ₚCH₂CH₂X

   tragende Nitril (oder das Gemisch solcher Nitrile) gegenüber dem mindestens eine dieser Gruppen tragenden Nitril (bzw. dem Gemisch solcher Nitrile) so im Ueberschuss eingesetzt werden muss, dass das erforderliche, oben definierte Verhältnis zwischen a) und b) erhalten wird.
- Durch Vermischen der im folgenden beschriebenen Komponenten 1 und 2.

Die Komponente 1 besteht aus mindestens einem 1,4-Diketopyrrolo-[3,4-c]-pyrrol, wie es unter a) definiert ist.

Die Komponente 2 besteht entweder aus mindestens einem 1,4-Diketopyrrolo-[3,4-c]-pyrrol, wie es unter b) definiert ist oder aus einem Gemisch aus mindestens einem 1,4-Diketopyrrolo-[3,4-c]-pyrrol, wie unter a) definiert und mindestens einem 1,4-Diketopyrrolo-[3,4-c]-pyrrol, wie unter b) definiert.

Das Vermischen der beiden Komponenten 1 und 2 erfolgt nach beliebigen, allgemein bekannten Methoden. Die Komponente 2 kann z.B. als feuchter Presskuchen oder als Pulver während der Synthese, der Rektristallisation oder der Filtration der Komponente 1 dieser letzteren beigemischt werden. Die Komponenten 1 und 2 können auch durch intensives Mischen bzw. Mahlen vermischt werden oder sie können dem zu färbenden hochmolekularen organischen Material zugegeben werden und während des Dispersionsprozesses vermischt werden.

Die erfindungsgemässen Pigmentzusammensetzungen können als Pigmente zum Färben von hochmolekularem organischem Material verwendet werden.

Hochmolekulare organische Materialien, die mit den erfindungsgemässen Pigmentzusammensetzungen gefärbt bzw. pigmentiert werden können, sind z.B. Celluloseether und -ester, wie Ethylcellulose, Nitrocellulose, Celluloseacetat, Cellulosebutyrat, natürliche Harze oder Kunstharze, wie Polymerisationsharze oder Kondensationsharze, wie Aminoplaste, insbesondere Harnstoff- und Melamin-Formaldehydharze, Alkyldharze, Phenoplaste, Polycarbonate, Polyolefine, Polystyrol, Polyvinylchlorid, Polytetrafluoroethylen, Polyamide, Polyurethane, Polyester, Polyether-ketone, Polyphenylenoxide, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Pigmentzusammensetzungen als Toner oder in Form von Präparaten einzusetzen. Bezogen auf das zu pigmentierende hochmolekulare organische Material kann man die erfindungsgemässen Pigmentzusammensetzungen in einer Menge von 0,01 bis 30 Gew.%, vorzugsweise von 0,1 bis 10 Gew.%, einsetzen.

Zum Pigmentieren von Lacken und Druckfarben werden die hochmolekularen organischen Materialien und die erfindungsgemässen Pigmentzusammensetzungen gegebenenfalls zusammen mit Zusatzstoffen, wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Die erhaltenen Färbungen, beispielsweise in Kunststoffen, Fasern, Lacken oder Drucken, zeichnen sich durch gute allgemeine Eigenschaften, wie hohe Farbstärke, gute Dispergierbarkeit, gute Ueberlackier-, Migrations-, Hitze-, Licht- und Wetterbeständigkeit, geringe Viskosität sowie durch einen guten Glanz aus.

Ferner weisen die erfindungsgemässen Pigmentzusammensetzungen im Vergleich zu den unmodiflzierten Basispigmenten verbesserte anwendungstechnische Eigenschaften in der Applikation auf, wie eine verbesserte Rheologie und Lagerbeständigkeit, geringere Trenneffekte wie Ausschwimmen bei der Mitverwendung von z.B. Weisspigmenten, und eine geringere Flockulationstendenz. Infolge der guten rheologischen Eigenschaften dieser Kompositionen ist es auch möglich, Anstrichstoffe und Lacke hoher Konzentration (sogenannte high loadings) herzustellen. Sie eignen sich demnach vorzugsweise zum Färben von Lacken, insbesondere für Metalleffektlackierungen.

Die oben beschriebene Komponente 2 kann allerdings auch selbst als Pigment zum einfärben der oben aufgeführten hochmolekularen organischen Materialien eingesetzt werden. Hierfür kann sie als Rohprodukt oder aber nach zweckmässiger Konditionierung/Nachbehandlung, beispielsweise wie oben für die erfindungsgemässen Pigmentzusammensetzungen bereits aufgeführt, eingesetzt werden.

Die nachfolgenden Beispiele erläutern die Erfindung.

Beispiel 1: 2,3 g Natrium werden in 55 ml tert.-Amylalkohol bis zur vollständigen Lösung unter Rückfluss kräftig gerührt. Man lässt die Temperatur auf 95-100°C sinken, gibt 6,38 g 4-[2-(Dimethylamino)-ethoxy]-benzonitril und danach, innerhalb von 45 Minuten, 7,73 g des Pyrrolinons der Formel 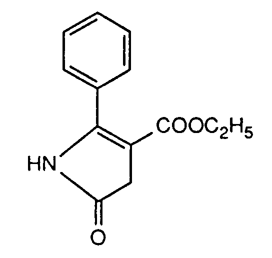 hinzu und rührt 2 Stunden bei 95-100°C weiter. Die Reaktionsmischung wird dann auf 40°C gekühlt und unter Rühren in eine Lösung von 6,7 g Essigsäure in 500 ml Methanol gegossen. Das erhaltene Produkt wird abfiltriert, nacheinander mit Methanol und Wasser gewaschen und über Nacht im Vakuum bei 90°C getrocknet. Man erhält 8,41 g (67 % d.Th.) des Produktes der Formel 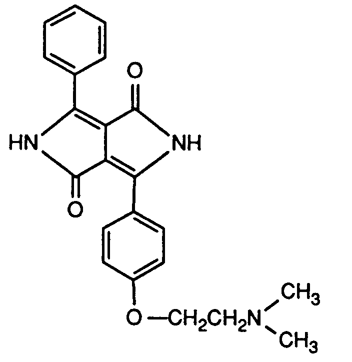 in Form eines gelbroten Pulvers.

| Analyse: | | | |
|---|---|---|---|
| Ber.: | C 70,38 % | H 5,64 % | N 11,19 % |
| Gef.: | C 70,11 % | H 5,64 % | N 11,17 % |

Beispiel 2: In einem Sulfierkolben werden 17,2 g 3,6-Diphenyl-1,4-diketopyrrolo-[3,4-c]-pyrrol als feuchter Presskuchen (29,1 %; 5,0 g Trockensubstanz) in 100 ml deionisiertem Wasser während einer Stunde bei Raumtemperatur gerührt. Anschliessend werden 0,24 g der nach Beispiel 1 hergestellten Verbindung als feuchter Presskuchen (54 %; 0,13 g Trockensubstanz) zugegeben und die Suspension wird 2 Stunden bei Raumtemperatur gerührt. Nach dem Filtrieren und Trocknen im Vakuum bei 80°C erhält man 5,2 g eines roten Pigments.

Beispiel 3: In einem 100 ml-Sulfierkolben werden unter Stickstoff 35 ml trockenes t-Amylalkohol und 1,38 g Natrium vorgelegt und bei 105°C unter starkem Rühren 17 Stunden bis zur vollständigen Umsetzung des Natriums durchmischt. Bei 100°C werden 4,9 g 4-(3-Dimethylaminopropoxy)-benzonitril und 2,6 g Benzonitril zugegeben und anschliessend während 80 Minuten 4,5 g Bernsteinsäurediisopropylester zugetropft. Nach 2 Stunden Rühren bei 110°C wird das Reaktionsgemisch auf Raumtemperatur abgekühlt und in einem 350 ml-Sulfierkolben in eine auf 0°C gekühlte Mischung aus 70 ml Methanol/Wasser 1:1 (Vol) und 3,43 ml Essigsäure eingetragen. Anschliessend wird die Mischung 2 Stunden bei Raumtemperatur gerühr, filtriert und der Rückstand wird mit Methanol und Wasser gewaschen und bei 90°C in einem Vakuumtrockenschrank getrocknet. Man erhält ein dunkelrotes Pigment. Die Massenspektroskopische Analyse (FD) ergibt folgende Daten: m/e 288, 389, 490.

Beispiel 4-16: wiederholt man Beispiel 3 unter Verwendung äquivalenter Mengen der entsprechenden Nitrile, dann erhält man die in Tabelle 1 aufgeführten Verbindungen.

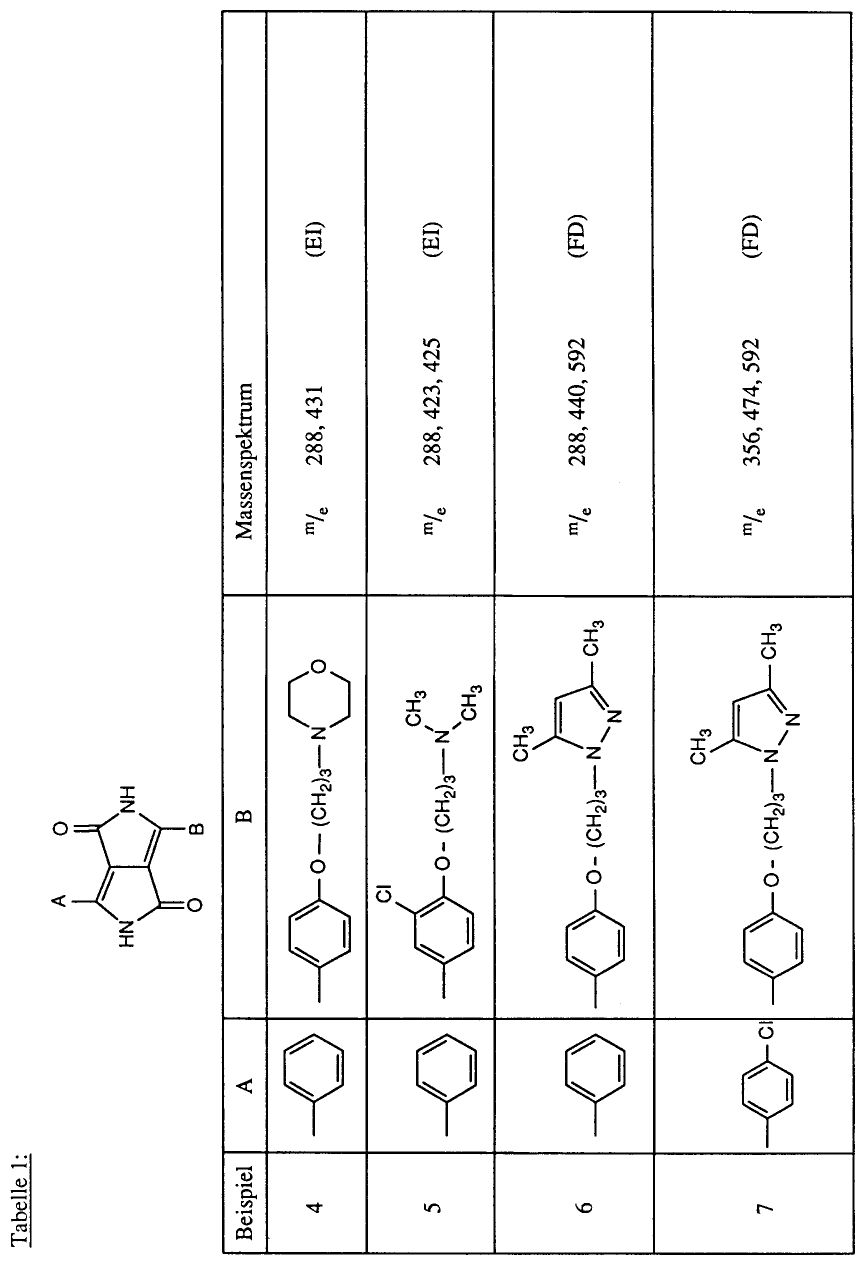

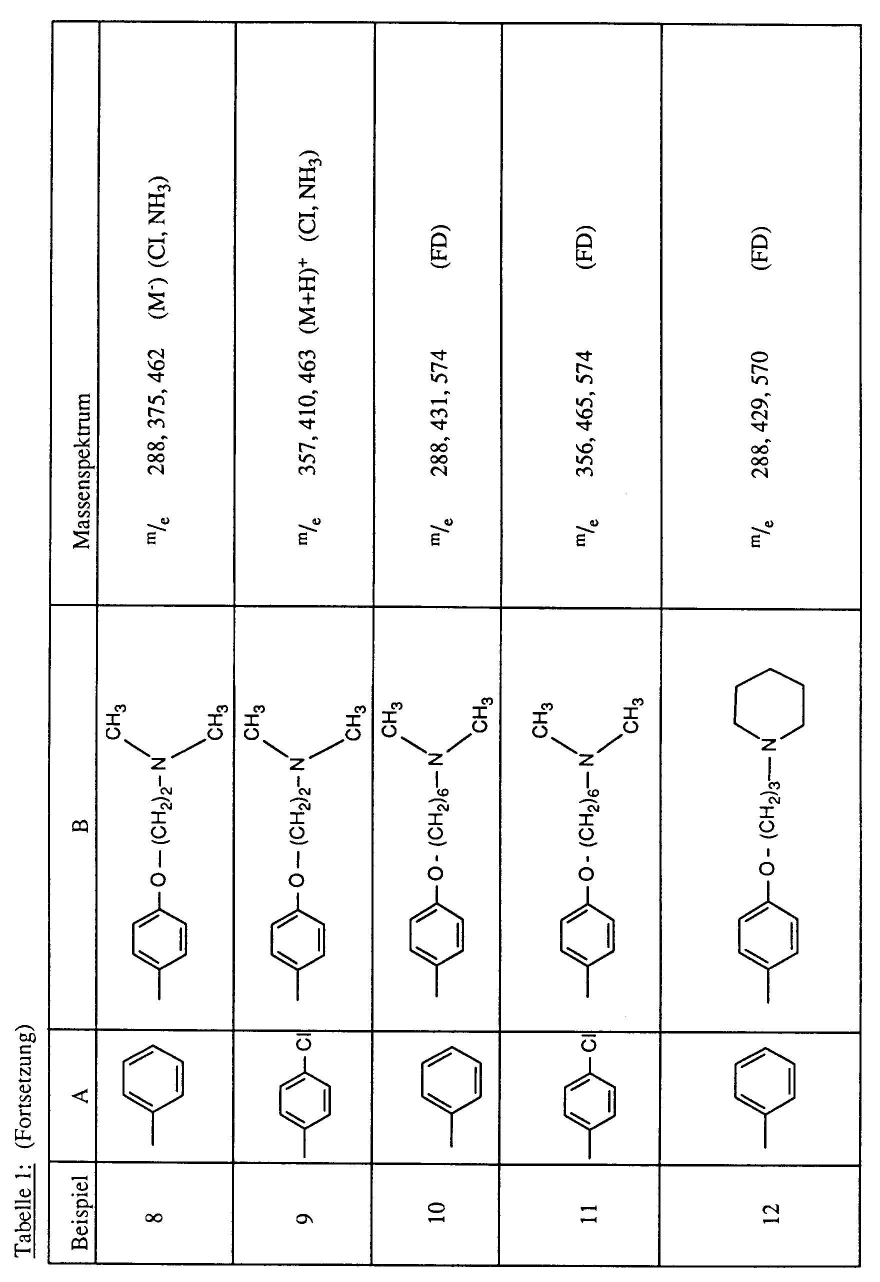

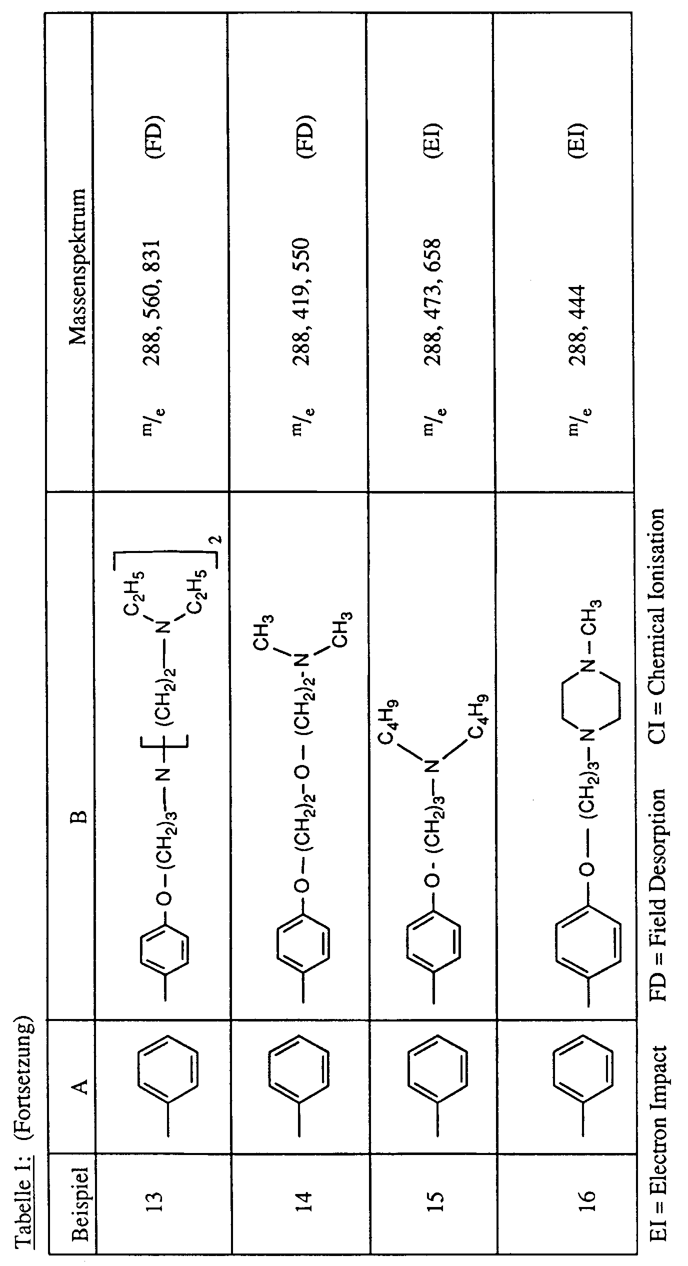

Beispiel 17 und 18: Wiederholt man Beispiel 3 in der Weise, dass man jeweils nur ein Nitril (entsprechend der in Tabelle 2 aufgeführten Reste A), aber in zweifacher äquivalenter Menge einsetzt, so erhält man die in Tabelle 2 aufgeführten Verbindungen.

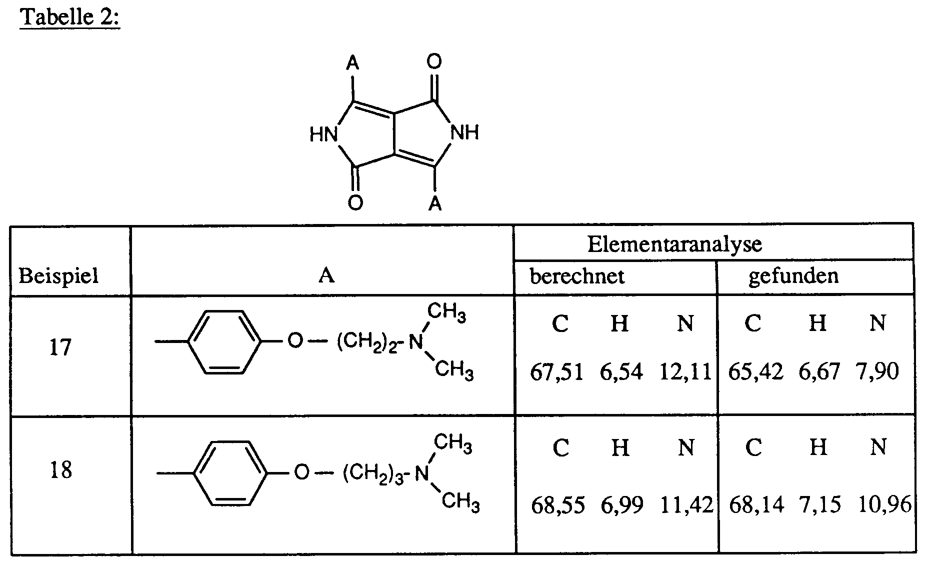

Beispiel 19: Zur Bestimmung des Fliessverhaltens wird das wie in Beispiel 2 beschrieben behandelte Pigment nach üblicher Methode in ein Alkyd-Lacksystem (®SETAL 84, Kunstharzfabrik Synthesis B.V., Holland; Festkörpergehalt: 70 Gew.%) eingearbeitet.

Das Fliessverhalten der Lackanreibung, welche 12 Gew.% Pigment und 54 Gew.% Gesamtfestkörper enthält und deren Pigment/Bindemittel-Verhältnis 0,3 beträgt, wird mit einem HAAKE-Viskosimeter ®ROTO-VISCO RV 12 ermittelt (Messtemperatur: 25°C, Mess-System: SV-SP, Scherbereich; D = 0-100 [1/s]).

Das gemäss Beispiel 2 erhaltene rote Pigment weist gegenüber unbehandeltem 3,6-Diphenyl-1,4-diketopyrrolo-[3,4-c]-pyrrol, neben ausgezeichneten koloristischen Eigenschaften, ein verbessertes rheologisches Verhalten auf.

Beispiel 20: Mit dem behandelten Pigment gemäss Beispiel 2 wird eine Offsetdruckfarbe im Dreiwalzenstuhl nach DIN 53238-12 hergestellt und zwar mit
20 Teilen Pigment und
80 Teilen Firnis bestehend aus
- 50 Gew.%: ölmodifiziertem phenolischem Firnis (®ALVCO 1407)
- 32 Gew.%: Terephthalsäurealkyd-Leinöl-Harz (100 % Festkörpergehalt; ®TERLON 3)
- 18 Gew.%: Mineralöl (Siedegrenze 157-214°C; ®SUNOCO OIL)

Die so erhaltene Druckfarbe zeichnet sich durch ausgezeichnete rheologische Eigenschaften aus und die damit auf Kunstdruckpapier erhaltenen Drucke durch überraschend gute coloristische Eigenschaften (farbstarke rote Nuance).

## Patentansprüche

1. 1,4-Diketopyrrolo[3,4-c]-pyrrole der Formel 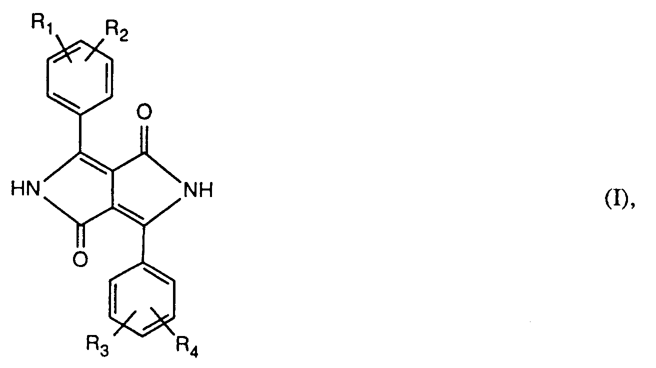 worin R₁, R₂, R₃ und R₄ unabhängig voneinander Wasserstoff, Cl, Br, CH₃, OCH₃, CN oder Phenyl bedeuten und mindestens einer der Reste R₁, R₂, R₃ und R₄ eine Gruppe
-O(CH₂)ₙX oder -O(CH₂CH₂O)ₚCH₂CH₂X
ist, worin
n eine Zahl von 2 bis 12 und
p eine Zahl von 1 bis 3 bedeuten,
X ein unsubstituierter oder ein- oder zweimal durch Methyl substituierter heterocyclischer Rest ausgewählt aus der Gruppe bestehend aus Imidazolyl, Pyrazolyl, Triazolyl, Piperazinyl, Pyridinyl, Pyrrolyl, Thiazolyl, Oxazolyl, Benzoxazolyl, Indolyl, Benzthiazolyl, Benzimidazolyl, Benztriazolyl, Morpholinyl, Piperidinyl und Pyrrolidinyl oder eine der Gruppen
-NR₅R₆
oder
-N[(CH₂)ₙ-NR₅R₆]₂ ist, worin
R₅ und R₆ unabhängig voneinander Wasserstoff, C₁-C₆-Alkyl oder C₅-C₆-Cycloalkyl sind.

2. 1,4-Diketopyrrolo-[3,4-c]-pyrrole gemäss Anspruch 1, der Formel I, worin mindestens einer der Reste R₁, R₂, R₃ oder R₄ eine Gruppe
-O(CH₂)ₙX oder -O(CH₂CH₂O)ₚCH₂CH₂X
ist, worin
n und p die in Anspruch 1 angegebene Bedeutung haben und
X ein heterocyclischer Rest ausgewählt aus der Gruppe bestehend aus Imidazolyl, Pyrazolyl, Triazolyl, Piperazinyl, Pyridinyl, Pyrrolyl, Thiazolyl, Oxazolyl, Morpholinyl, Piperidinyl und Pyrrolidinyl, oder eine Gruppe
-NR₅R₆
mit der in Anspruch 1 angegebenen Bedeutung ist.

3. 1,4-Diketopyrrolo-[3,4-c]-pyrrole gemäss Anspruch 1, der Formel I, worin einer oder zwei der Reste R₁, R₂, R₃ oder R₄ eine Gruppe
-O(CH₂)ₙX oder -O(CH₂CH₂O)ₚCH₂CH₂X
ist, worin
n eine Zahl von 2 bis 6 und
p die Zahl 1 oder 2 bedeuten und
X ein heterocyclischer Rest ausgewählt aus der Gruppe bestehend aus Imidazolyl, Pyrazolyl, Triazolyl, Piperazinyl, Pyridinyl, Pyrrolyl, Thiazolyl, Oxazolyl, Morpholinyl, Piperidinyl und Pyrrolidinyl,
oder eine Gruppe
-NR₅R₆
bedeutet, worin R₅ und R₆ unabhängig voneinander Wasserstoff, Methyl oder Ethyl bedeuten.

4. 1,4-Diketopyrrolo-[3,4-c]-pyrrole gemäss Anspruch 3, dadurch gekennzeichnet, dass X ein heterocyclischer Rest ausgewählt aus der Gruppe bestehend aus Imidazolyl, Pyrazolyl, Morpholinyl, Piperidinyl, Pyrrolidinyl und Triazolyl oder eine Gruppe -NR₅R₆ ist, worin R₅ und R₆ die in Anspruch 3 angegebene Bedeutung haben.

5. 1,4-Diketopyrrolo-[3,4-c]-pyrrole gemäss Anspruch 1, der Formel I, worin einer der Reste R₃ oder R₄ eine Gruppe
-O(CH₂)ₙNR₅R₆ oder -O(CH₂)ₙX
ist, worin n eine Zahl von 2 bis 4 bedeutet und R₅ und R₆ gleich sind und Wasserstoff, Methyl oder Ethyl und X Morpholinyl, Piperidinyl oder Pyrrolidinyl bedeuten.

6. Pigmentzusammensetzungen enthaltend
a) 80-99,9 Gew.% mindestens eines 1,4-Diketopyrrolo-[3,4-c]-pyrrols der Formel I, worin R₁, R₂, R₃ und R₄ unabhängig voneinander Wasserstoff, Cl, Br, CH₃, OCH₃, CN oder Phenyl bedeuten, und
b) 0,1-20 Gew.% mindestens eines 1,4-Diketopyrrolo-[3,4-c]-pyrrols der Formel I, gemäss Anspruch 1.

7. Hochmolekulares organisches Material enthaltend als Farbmittel eine Pigmentzusammensetzung gemäss Anspruch 6.

8. Hochmolekulares organisches Material gemäss Anspruch 7, dadurch gekennzeichnet, dass es ein Lack oder eine Druckfarbe ist.

## Claims

1. A 1,4-diketopyrrolo[3,4-c]pyrrole of formula 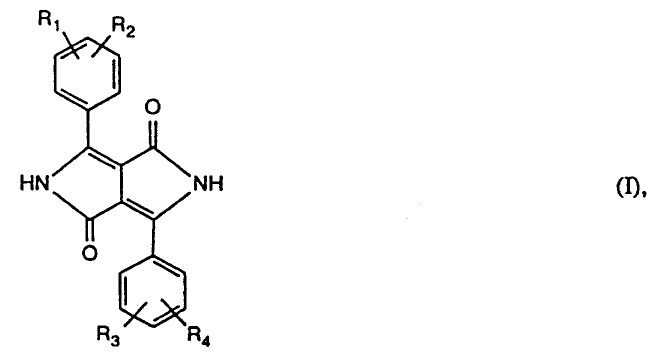 wherein R₁, R₂, R₃ and R₄ are each independently of one another hydrogen, Cl, Br, CH₃, OCH₃, CN or phenyl, and at least one of the radicals R₁, R₂, R₃ and R₄ is a group
-O(CH₂)ₙX or -O(CH₂CH₂O)ₚCH₂CH₂X
wherein
n is a number from 2 to 12, and
p is a number from 1 to 3,
X is a heterocyclic radical selected from the group consisting of imidazolyl, pyrazolyl, triazolyl, piperazinyl, pyridinyl, pyrrolyl, thiazolyl, oxazolyl, benzoxazolyl, indolyl, benzothiazolyl, benzimidazolyl, benzotriazolyl, morpholinyl, piperidinyl and pyrrolidinyl, which radical is unsubstituted or substituted by one or two methyl groups, or is a group -NR₅R₆
or
-N[(CH₂)ₙ-NR₅R₆]₂, wherein
R₅ and R₆ are each independently of the other hydrogen, C₁-C₆alkyl or C₅-C₆cycloalkyl.

2. A 1,4-diketopyrrolo[3,4-c]pyrrole of formula I according to claim 1, wherein at least one of the radicals R₁, R₂, R₃ or R₄ is a group
-O(CH₂)ₙX or -O(CH₂CH₂O)ₚCH₂CH₂X
wherein
n and p are as defined in claim 1, and X is a heterocyclic radical selected from the group consisting of imidazolyl, pyrazolyl, triazolyl, piperazinyl, pyridinyl, pyrrolyl, thiazolyl, oxazolyl, morpholinyl, piperidinyl and pyrrolidinyl, or is a group
-NR₅R₆
as defined in claim 1.

3. A 1,4-diketopyrrolo[3,4-c]pyrrole of formula I according to claim 1, wherein one or two of the radicals R₁, R₂, R₃ or R₄ is a group
-O(CH₂)ₙX or -O(CH₂CH₂O)ₚCH₂CH₂X
wherein
n is a number from 2 to 6 and
p is the number 1 or 2, and
X is a heterocyclic radical selected from the group consisting of imidazolyl, pyrazolyl, triazolyl, piperazinyl, pyridinyl, pyrrolyl, thiazolyl, oxazolyl, morpholinyl, piperidinyl and pyrrolidinyl, or is a group
-NR₅R₆
wherein R₅ and R₆ are each independently of the other hydrogen, methyl or ethyl.

4. A 1,4-diketopyrrolo[3,4-c]pyrrole according to claim 3, wherein X is a heterocyclic radical selected from the group consisting of imidazolyl, pyrazolyl, morpholinyl, piperidinyl, pyrrolidinyl and triazolyl, or is a group -NR₅R₆, wherein R₅ and R₆ are as defined in claim 3.

5. A 1,4-diketopyrrolo[3,4-c]pyrrole of formula I according to claim 1, wherein one of the radicals R₃ or R₄ is a group
-O(CH₂)ₙNR₅R₆ or -O(CH₂)ₙX
wherein n is a number from 2 to 4 and R₅ and R₆ are identical and are hydrogen, methyl or ethyl, and X is morpholinyl, piperidinyl or pyrrolidinyl.

6. A pigment composition comprising
a) 80-99.9 % by weight of at least one 1,4-diketopyrrolo[3,4-c]pyrrole of formula I, wherein R₁, R₂, R₃ and R₄ are each independently of one another hydrogen, Cl, Br, CH₃, OCH₃, CN or phenyl, and
b) 0.1-20 % by weight of at least one 1,4-diketopyrrolo[3,4-c]pyrrole of formula I according to claim 1.

7. High molecular weight organic material comprising as colourant a pigment composition according to claim 6.

8. High molecular weight organic material according to claim 7, which is a paint or a printing ink.

## Revendications

1. 1,4-Dicétopyrrolo-[3,4-c]-pyrroles de formule 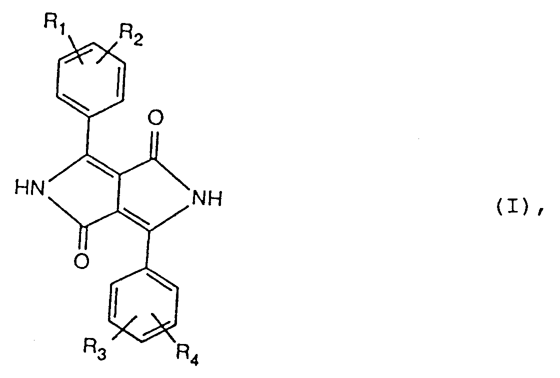 dans laquelle R₁, R₂, R₃ et R₄, indépendamment les uns des autres, représentent l'hydrogène, Cl, Br, CH₃, OCH₃, CN ou phényle et au moins un des radicaux R₁, R₂, R₃ et R₄ est groupe
-O(CH₂)ₙX ou -O(CH₂CH₂O)ₚCH₂CH₂X
dans lequel
n est un nombre de 2 à 12 et
p est un nombre de 1 à 3,
X représente un radical hétérocyclique non substitué ou substitué une ou deux fois par méthyle pris dans le groupe comportant imidazolyle, pyrazolyle, triazolyle, pipérazinyle, pyridinyle, pyrrolyle, thiazolyle, oxazolyle, benzoxazolyle, indolyle, benzothiazolyle, benzimidazolyle, benzotriazolyle, morpholinyle, pipéridinyle et pyrrolidinyle ou un des groupes -NR₅R₆ ou -N[(CH₂)ₙ-NR₅R₆]₂, dans lesquels
R₅ et R₆, indépendamment l'un de l'autre, représentent l'hydrogène, alkyle en C₁-C₆ ou cycloalkyle en C₅-C₆.

2. 1,4-Dicétopyrrolo-[3,4-c]-pyrroles selon la revendication 1 de formule I où au moins l'un des radicaux R₁, R₂, R₃ et R₄ représentent un groupe
-O(CH₂)ₙX ou -O(CH₂CH₂O)ₚCH₂CH₂X
dans lequel
n et p ont la signification donnée dans la revendication 1 et
X représente un radical hétérocyclique pris dans le groupe comportant imidazolyle, pyrazolyle, triazolyle, pipérazinyle, pyridinyle, pyrrolyle, thiazolyle, oxazolyle, morpholinyle, pipéridinyle et pyrrolidinyle ou un groupe -NR₅R₆ ayant la signification donnée dans la revendication 1.

3. 1,4-Dicétopyrrolo-[3,4-c]-pyrroles selon la revendication 1 de formule I où un ou deux des radicaux R₁, R₂, R₃ ou R₄ représente un groupe
-O(CH₂)ₙX ou -O(CH₂CH₂O)ₚCH₂CH₂X
dans lequel
n est un nombre de 2 à 6 et
p vaut 1 ou 2 et
X représente un radical hétérocyclique pris dans le groupe comportant imidazolyle, pyrazolyle, triazolyle, pipérazinyle, pyridinyle, pyrrolyle, thiazolyle, oxazolyle, morpholinyle, pipéridinyle et pyrrolidinyle ou un groupe -NR₅R₆ dans lequel R₅ et R₆, indépendamment l'un de l'autre, représentent l'hydrogène, méthyle ou éthyle.

4. 1,4-dicétopyrrolo-[3,4-c]-pyrroles selon la revendication 3 caractérisés en ce que X est un radical hétérocyclique pris dans le groupe comportant imidazolyle, pyrazolyle, morpholinyle, pipéridinyle, pyrrolidinyle et triazolyle ou d'un groupe -NR₅R₆ dans lequel R₅ et R₆ ont la signification donnée dans la revendication 3.

5. 1,4-Dicétopyrrolo-[3,4-c]-pyrroles selon la revendication 1 de formule I où un des radicaux R₃ ou R₄ représente un groupe
-O(CH₂)ₙNR₅R₆ ou -O(CH₂)ₙX
où n est un nombre de 2 à 4 et R₅ et R₆ sont identiques et représentent l'hydrogène, méthyle ou éthyle et X représente morpholinyle, pipéridinyle ou pyrrolidinyle.

6. Compositions pigmentaires contenant
a) de 80 à 99,9 % en poids d'au moins un 1,4-dicétopyrrolo-[3,4-c]-pyrrole de formule I dans lequel R₁, R₂, R₃ et R₄, indépendamment les uns des autres, représentent l'hydrogène, Cl, Br, CH₃, OCH₃, CN ou phényle, et
b) de 0,1 à 20 % en poids d'au moins un 1,4-dicétopyrrolo-[3,4-c]-pyrrole de formule I selon la revendication 1.

7. Matière organique de haut poids moléculaire contenant comme colorant une composition pigmentaire selon la revendication 6.

8. Matière organique de haut poids moléculaire selon la revendication 7, caractérisée en ce qu'elle est un vernis ou une encre d'imprimerie.
